# EUROPÄISCHE PATENTSCHRIFT

(11) **EP 0 834 300 B1**
(45) Veröffentlichungstag und Bekanntmachung des Hinweises auf die Patenterteilung: **14.08.2002**
(21) Anmeldenummer: 97116523.8
(22) Anmeldetag: 23.09.1997
(51) Int. Cl.: A61K 7/00, A61K 7/06, A61K 7/48

(54) **Verwendung von carboxylgruppenhaltigen Polysiloxanen in kosmetischen Formulierungen**
Use of carboxylgroups-containing polysiloxanes in cosmetical formulations
Utilisation de polysiloxanes à groupes carboxyliques dans des formulations cosmétiques

(30) Priorität: 27.09.1996 DE 19639669
(43) Veröffentlichungstag der Anmeldung: 08.04.1998
(73) Patentinhaber: BASF AKTIENGESELLSCHAFT, 67056 Ludwigshafen (DE)
(72) Erfinder: Hössel, Peter, Dr., 67105 Schifferstadt (DE); Kneip, Michael, Dr., 67069 Ludwigshafen (DE); Greif, Norbert, Dr., 67273 Bobenheim (DE)

(56) Entgegenhaltungen:
- FR-A- 2 443 476
- H.J.KOLLMEIER,T.H.GOLDSCHMIDT: "Organo-polysiloxanes for cosmetic formulations" SOAP,PERFUMERY % COSMETICS, Bd. 72, Nr. 7, 1.Juli 1985, LONDO, GB, Seiten 371-377, XP002054122

## Beschreibung

Die vorliegende Erfindung betrifft die Verwendung von carboxylgruppenhaltigen Polysiloxanen in kosmetischen Formulierungen, insbesondere in Haut- und Haarpflegemitteln.

DE-A-3340708 beschreibt quaternierte Polysiloxanpolymere und diese enthaltende kosmetische Mittel wie Shampoos und Spüllotionen. Die haarpflegenden Eigenschaften dieser Polymere sind jedoch nicht ausreichend, insbesondere bei der Pflege von stark geschädigtem Haar nach Haarbleichen.

EP-A-324345 beschreibt ein Verfahren zum Hydrophobieren von Leder und Pelzen mit einem carboxylgruppenhaltigen Polysiloxan, bei dem die Carboxylgruppen in neutralisierter Form vorliegen.

Es bestand die Aufgabe, auf der Basis von Polysiloxanen ein Polymer für kosmetische Formulierungen zu entwickeln, das hinsichtlich seiner Haut- und Haarpflegewirkung die Materialien des Standes der Technik übertrifft.

Gefunden wurde die Verwendung von carboxylgruppenhaltigen Polysiloxanen, deren Carboxylgruppen zu 0 bis 50 Mol.-% amidiert sind, in kosmetischen Formulierungen.

Die erfindungsgemäß verwendeten carboxylgruppenhaltigen Polysiloxane sind dem Fachmann an sich bekannt. Besonders geeignet sind Polysiloxane, bei denen die restlichen Valenzen des Siliciums durch aliphatische oder aromatische Kohlenwasserstoffreste, insbesondere Methyl, aber auch Ethyl, Propyl oder Phenyl, abgesättigt sind und die von ihrer Herstellung her über diese Kohlenwasserstoffreste Carbonsäuren oder Carbonsäurenanhydridgruppen endständig und/oder in den Seitenketten im Molekül eingebaut enthalten. Die erfindungsgemäß verwendeten carboxylgruppenhaltigen Polysiloxane enthalten bevorzugt im Durchschnitt 2 bis 10 Carboxylgruppen pro Molekül.

Besonders bevorzugte carboxylgruppenhaltige Polysiloxane sind Dimethylpolysiloxane und Methyl-propyl-polysiloxane, die endständig Carbonsäureanhydridgruppen aufweisen. Ganz besonders bevorzugt sind Dimethylpolysiloxane und Methyl-propyl-polysiloxane, die endständig Bernsteinsäureanhydridgruppen aufweisen.

Zur Amidierung bzw. anschließenden Neutralisation der vorhandenen Carbonsäuregruppen bzw. zur Hydrolyse und Neutralisation vorhandener Säureanhydridgruppen werden die Polysiloxane zweckmäßig in wäßriger oder alkoholischer Lösung mit einer Base oder einem Amin umgesetzt bis sich ein pH-Wert von 7 - 10, bevorzugt 7 - 8,5 einstellt. Es kann auch eine Mischung aus Amin und einer Base wie Alkalihydroxid verwendet werden.

Die Amidierung und Neutralisation der Carboxylgruppen kann aber > auch getrennt durchgeführt werden. Dabei wird zunächst ein Teil der Carboxylgruppen mit einem Amin oder einem Gemisch verschiedener Amine in das entsprechende Amid bzw. die entsprechenden Amide überführt und anschließend werden die verbliebenen freien Carboxylgruppen mit einer Base, einem Amin, einem Gemisch verschiedener Amine oder einem Gemisch aus den genannten Komponenten ganz oder teilweise neutralisiert.

Die Umsetzung kann auch direkt mit dem Amin ohne Lösungsmittel durchgeführt werden.

Besonders bevorzugt werden die Carbonsäureanhydridgruppen tragenden Polysiloxane in Gegenwart von Aminen, Aminoalkoholen oder deren Gemischen umgesetzt, so daß die Anydride unter Ringöffnung amidiert werden.

In der Amidierungsreaktion werden 0 bis 50 Mol.-% der Carboxylgruppen in das Amid überführt. Bevorzugt werden 5 bis 50 Mol.-%, besonders bevorzugt 20 bis 50 Mol.-% und ganz besonders bevorzugt 40 bis 50 Mol.-% in das Amid überführt.

In der anschließenden Neutralisation der verbliebenen freien Carboxylgruppen mit Base oder Amin werden 0 bis 100 Mol.-% der freien Carboxylgruppen neutralisiert. Dabei sollte der Anteil des Amins am Neutralisationsmittel zweckmäßigerweise so groß sein, daß mindestens 50 Mol-%, bevorzugt mindestens 70 Mol-% und besonders bevorzugt 80-100 Mol-% der freien Carboxylgruppen mit Amin neutralisiert werden. Die Neutralisation der carboxylgruppenhaitigen Polysiloxane kann auch ohne vorheriger Amidierung erfolgen.

Als Amin können alle primären, sekundären oder tertiären Amine oder Mischungen hieraus eingesetzt werden. Bevorzugt sind primäre oder tertiäre Amine, da die sekundären Amine nachteiligerweise Nitrosamine bilden können. Die Reaktion ist aber auch prinzipiell mit sekundären Aminen durchführbar. Darüber hinaus sind auch aminogruppenhaltige Alkohole wie Mono-, Di- oder Trialkanolamin mit 2-6 C-Atomen im Alkanolrest, beispielsweise Mono-, Di- oder Triethanolamin gut geeignet.

Besonders gut geeignete Amine sind Morpholin, Ethanolamin, 2-Amino-2-methyl-propanol-1 sowie Mischungen daraus, insbesondere Ethanolamin, 2-Amino-2-methyl-propanol-1 und Mischungen davon mit anderen Aminen und Basen.

Die Amidierung und/oder Neutralisation der Carboxylgruppen mit den Aminen erfolgt üblicherweise bei einer Temperatur von 20-130, bevorzugt bei 40-100°C.

Gegenstand der Erfindung ist die Verwendung solcher carboxylgruppenhaltiger Polysiloxane für kosmetische Formulierungen, insbesondere für Haut- und Haarpflegemittel.

Bevorzugt verwendet werden die carboxylgruppenhaltigen Polysiloxane als Haarfestiger , als Conditioner, als Verdickungsmittel sowie als Hilfsmittel bei der Haarfärbung, dem Haarbleichen und beim Dauerwellverfahren.

Besonders vorteilhaft werden die carboxylgruppenhaltigen Polysiloxane in Form von Emulsionen eingesetzt. Diese Emulsionen können alle in der Kosmetik üblichen Wirk- und Hilfsstoffe enthalten.

Üblicherweise werden emulgierende Substanzen mit Wasser, Ölen, Fetten und ggf. Fettalkoholen, Fettsäuren, Konservierungsmittel, Polymeren, Konsistenzgebern und gelbildenden Stoffen zusammengebracht.

Als Wirkstoffe können Lichtschutzmittel, Bleichmittel, Haarfärbemittel, Dauerwellenmittel, kationische Tenside, anionische Tenside, Feuchthaltemittel, Harnstoff, Vitamine, Panthenol, Panthenolethylether und/oder Bisabolol enthalten sein.

Ein weiterer Gegenstand der Erfindung ist die Verwendung der carboxylgruppenhaltigen Polysiloxane in Mundpflegemitteln und bei der Fuß- und Fingernagelpflege.

Für die erfindungsgemäße Verwendung können neben den carboxylgruppenhaltigen Polysiloxanen auch alle normalerweise in kosmetischen Mitteln verwendeten Bestandteile eingesetzt werden, insbesondere ionische und nichtionische oberflächenaktive Mittel, Schaumsynergisten, Schaumstabilisatoren, opakmachende Mittel, Sequestriermittel, Verdickungsmittel, Emulgatoren, weichmachende Mittel, Konservierungsmittel, Proteinderivate, natürliche Substanzen, Farbstoffe, Parfüms und ggf. weitere Polymere oder Hilfsstoffe.

### Beispiel 1-4

### Herstellung von Polymer 1:

Zu 167 g Silikonöl IM 86 (Wacker) werden bei 60 °C 30 g Morpholin und 3 g Ölsäure zugegeben und 60 Min. bei 60 °C gerührt. Das IR-Spektrum des Reaktionsproduktes zeigt keine Anhydrid-Bande bei 1780 nm mehr. Ca. 800 ml Wasser werden zugegeben, mit einem Ultraturrax voremulgiert und dann über ein Hochdruckemulgiergerät bei 150 bar emulgiert.

### Herstellung von Polymer 2: Ethanolamin als Aminkomponente

Zu 211,2 g Silikonöl IM 86 werden unter Rühren bei einer Temperatur < 70 °C 24,4 g Ethanolamin zugetropft. Anschließend wird bei o.g. Temperatur für zwei Stunden nachgerührt. Das erhaltene Reaktionsgemisch wird mit 2,65 g Ölsäure versetzt und zur Herstellung einer Emulsion verwendet. Hierzu werden zu 200 g des Produktes 800 ml Wasser zugefügt, die Mischung unter Verwendung eines Ultraturrax voremulgiert und dann mittels eines Hochdruckemulgiergerätes bei 150 bar emulgiert.

### Herstellung von Polymer 3: 2-Amino-2-methyl-propanol-1 als Aminkomponente

Die Herstellung erfolgt wie für Polymer 2 beschrieben, indem 209,2 g Silikonöl IM 86, 36,3 g 2-Amino-2-methyl-propanol-1 und 2,65 g Ölsäure umgesetzt werden.

### Herstellung von Polymer 4: Morpholin und 2-Amino-2-methyl-propanol-1 als Aminkomponenten

Zu 182,1 g Silikonöl IM 86 werden unter Rühren bei einer Temperatur < 70 °C innerhalb von 15 Minuten 10,8 g Morpholin zugetropft und der Ansatz 30 Minuten bei 60 °C nachgerührt. Das IR-Spektrum der Reaktionsmischung zeigt dann keine Anhydridbande bei 1780 cm⁻¹ mehr. Das erhaltene Reaktionsprodukt wird mit 16 g 2-Amino-2-methyl-propanol-1 versetzt und 1,5 Stunden bei 60 °C gerührt. Nach Zusatz von 2,4 g Ölsäure und 845 g Wasser wird das Produkt wie bereits beschrieben zur Herstellung einer Emulsion verwendet.

### Verfahrensvariante zur Herstellung der erfindungsgemäßen Verbindungen

Die erfindungsgemäßen Verbindungen können auch nach anderen als in den Beispielen angegebenen Verfahren hergestellt werden. Eine vorteilhafte Verfahrensvariante besteht zum Beispiel darin, die Aminkomponente zusammen mit Ölsäure in wäßriger Lösung vorzulegen und bei einer Temperatur < 70 °C das Anhydrid-funktionalisierte Polysiloxan zuzutropfen. Die Weiterverarbeitung zur Emulsion nach beendeter Umsetzung erfolgt dann analog zur bereits beschriebenen Vorgehensweise.

### Beispiel 5

### Herstellung von zwei Emulsionen

Es wurden die beiden Emulsionen 1 und 2 hergestellt und ihre haarpflegenden Eigenschaften ermittelt.

| Emulsion 1 | | |
|---|---|---|
| | g | INCI-Bezeichnung |
| Phase A: | 1,00 | Siliconderivat (Wirkstoff) |
| | 1,50 | Ceteareth-25 |
| | 1,50 | Ceteareth-6 and Stearyl Alcohol |
| | 6,00 | Cetearyl Octanoate |
| | 3,00 | Cetearyl Alcohol |
| | | |
| Phase B: | 2,00 | Propylene Glycole |
| | 0,30 | Imidazolidinyl Urea |
| | 0,10 | Benzyl Alcohol, Methylchloroisothiazolinone, Methylisothiazolinone |
| | ad 100,00 g | Wasser |

| Emulsion 2 | | |
|---|---|---|
| | g | INCI-Bezeichnung |
| Phase A: | 1,50 | Ceteareth-25 |
| | 1,50 | Ceteareth-6 and Stearyl Alcohol |
| | 6,00 | Cetearyl Octanoate |
| | 3,00 | Cetearyl Alcohol |
| | | |
| Phase B: | 1,00 | Siliconderivat (Wirkstoff) |
| | 2,00 | Propylene Glycole |
| | 0,30 | Inidazolidinyl Urea |
| | 0,10 | Benzyl Alcohol, Methylchloroisothiazolinone, Methylisothiazolinone |
| | ad 100,00 g | Wasser |

Phase A und B wurden getrennt unter Rühren auf 80 °C erhitzt. Phase A wurde unter Rühren in Phase B gegeben und abkühlt.

Der pH-Wert wurde nach der Herstellung mit Citronensäure eingestellt.

### Prüfungen:

Naßkämmbarkeit subjektive Beurteilung von ausgebildetem erfahrenem Friseur- und Laborpersonal an Haarsträhnen.
Notenskala: 1 (sehr gut), 2 (gut) und 3 (schlecht) Doppelbestimmung von zwei Personen an gebleichten Haarsträhnen mit mitteleuropäischem Haar; Breite 6 cm, Länge 25 cm.

Kämmkraftabnahme: Prüfung der Naßkämmbarkeit an einer Zug/Druck-Prüfmaschine. Messung der Abnahme der Kämmkraft im Vergleich zu einer unbehandelten Haarsträhne. Mittelwert aus 15 Einzelmessungen; Haare wie oben.

statische Aufladung: subjektive Beurteilung von ausgebildetem erfahrenem Friseur- und Laborpersonal nach 10-maligem Kämmen der oben genannten Haarsträhnen im trockenen Zustand im Klimaraum bei 50 % relativer Feuchte.

### Notenskala: 1 (keine); 2 (schwach); 3 (stark)

Griff des trockenen Haares subjektive Beurteilung von ausgebildetem erfahrenem Friseur- und Laborpersonal.

### Notenskala: 1 (sehr zart), 2 (zart), 3 (rauh)

**Tabelle 1:**

| Ergebnisse der anwendungstechnischen Prüfungen | | | | | | |
|---|---|---|---|---|---|---|
| Polymer Nr. | Emulsion Nr. | pH-Wert | Naßkämmbarkeit (Note) | Kämmkraftabnahme in % | statische Auflad ung | Griff (Note) |
| 1 | 2 | 2 - 3 | 1 - 2 | 54 | 1 | 2 |
| 2 | 2 | 2 - 3 | 1 -2 | 69 | 1 | 2 |
| 3 | 2 | 6 - 7 | 1 | 63 | 1 | 1 |
| 3 | 2 | 2 - 3 | 1 | 64 | 1 | 1 |
| 4 | 2 | 6 - 7 | 1 - 2 | - | 1 | 2 |
| 4 | 2 | 2 - 3 | 1 - 2 | - | 1 | 2 |

| Vergleichsbeispiele: | | | | | | |
|---|---|---|---|---|---|---|
| ohne Polymer | ohne Polymer | 2 - 3 | 2 - 3 | 0 | 3 | 3 |
| Quaternium-80 | 1 | 2 - 3 | 2 - 3 | 25 | 3 | 3 |
| Dimethicone Copolyol | 1 | 2 - 3 | 2 - 3 | 16 | 3 | 3 |
| Polyquaternium-11 | 2 | 2 - 3 | 2 | 36 | 3 | 2 |
| Dimethicone Propyl PG-Betain | 1 | 2 - 3 | 3 | 11 | 3 | 3 |
| Dimethicone Propyl PG-Betain | 2 | 2 - 3 | 3 | 17 | 3 | 3 |

## Patentansprüche

1. Verwendung von carboxylgruppenhaltigen Polysiloxanen, die hergestellt sind aus Dimethylpolysiloxanen oder Methyl-propyl-polysiloxanen, welche endständig Bernsteinsäureanhydridgruppen aufweisen, und deren Säureanhydridgruppen anschließend hydrolysiert und neutralisiert wurden, wobei die Carboxylgruppen zu 0 bis 50 Mol-% amidiert sind, in kosmetischen Formulierungen.

2. Verwendung nach Anspruch 1, **dadurch gekennzeichnet, daß** zur Amidierung der Carboxylgruppen der Polysiloxane Amine eingesetzt werden.

3. Verwendung nach Anspruch 1, **dadurch gekennzeichnet, daß** zur Amidierung der Carboxylgruppen der Polysiloxane 2-Amino-2-methyl-propanol-1, Ethanolamin, Morpholin und/oder Triethanolamin eingesetzt wird.

4. Verwendung nach einem der vorstehenden Ansprüche, **dadurch gekennzeichnet, daß** die nichtamidierten freien Carboxylgruppen ganz oder teilweise neutralisiert sind.

5. Verwendung nach einem der vorstehenden Ansprüche, **dadurch gekennzeichnet, daß** die carboxylgruppenhaltigen Polysiloxane Dimethylpolysiloxane sind.

6. Verwendung nach einem der vorstehenden Ansprüche als Hautund Haarpflegemittel.

7. Verwendung nach einem der vorstehenden Ansprüche in Form von Emulsionen.

8. Verwendung nach Anspruch 6 oder 7 in Shampoos.

9. Verwendung nach Anspruch 6 oder 7 in Haarfestigern.

10. Verwendung nach Anspruch 6 oder 7 als Hilfsmittel vor, während oder nach der Haarfärbung, dem Haarbleichen oder dem Dauerwellverfahren.

11. Verwendung nach Anspruch 1 bis 5 zur Mundpflege.

12. Verwendung nach Anspruch 1 bis 5 zur Finger- und Fußnagelpflege.

13. Kosmetische Formulierungen enthaltend carboxylgruppenhaltige Polysiloxane, die hergestellt sind aus Dimethylpolysiloxanen oder Methyl-propyl-polysiloxanen, welche endständig Bernsteinsäureanhydridgruppen aufweisen, und deren Säureanhydridgruppen anschließend hydrolysiert und neutralisiert wurden, wobei die Carboxylgruppen zu 0 bis 50 Mol-% amidiert sind.

## Claims

1. The use in cosmetic formulations of a carboxyl-containing polysiloxane prepared from dimethylpolysiloxanes or methylpropylpolysiloxanes which contain terminal succinic anhydride groups and whose acid anhydride groups have been subsequently hydrolyzed and neutralized, from 0 to 50 mol-% of the carboxyls having been amidated.

2. The use as claimed in claim 1, wherein amines are employed for amidating the polysiloxane carboxyls.

3. The use as claimed in claim 1, wherein 2-amino-2-methyl-1-propanol, ethanolamine, morpholine and/or triethanolamine is employed for amidating the polysiloxane carboxyls.

4. The use as claimed in any of the above claims, wherein some or all of the nonamidated free carboxyls are neutralized.

5. The use as claimed in any of the above claims, wherein the carboxyl-containing polysiloxane is a dimethylpolysiloxane.

6. The use as claimed in any of the above claims as skincare and haircare products.

7. The use as claimed in any of the above claims in the form of an emulsion.

8. The use as claimed in claim 6 or 7 in shampoos.

9. The use as claimed in claim 6 or 7 in hairsetting products.

10. The use as claimed in claim 6 or 7 as an auxiliary prior to, during or after coloring, bleaching or permanently waving the hair.

11. The use as claimed in any of claims 1 to 5 for oral care.

12. The use as claimed in any of claims 1 to 5 for nailcare for the fingers and toes.

13. A cosmetic formulation comprising a carboxyl-containing polysiloxane prepared from dimethylpolysiloxanes or methylpropylpolysiloxanes which contain terminal succinic anhydride groups and whose acid anhydride groups have been subsequently hydrolyzed and neutralized, from 0 to 50 mol-% of the carboxyls having been amidated.

## Revendications

1. Utilisation de polysiloxanes contenant des groupes carboxyle, qui ont été préparés à partir de diméthylpolysiloxanes ou de méthylpropylpolysiloxanes à groupes terminaux anhydride succinique et dont les groupes anhydride ont ensuite été hydrolysés et neutralisés, les groupes carboxyle étant amidés en proportions de 0 à 50 mol %, dans des compositions cosmétiques.

2. Utilisation selon la revendication 1, **caractérisée par le fait que**, pour l'amidation des groupes carboxyle des polysiloxanes, on a utilisé des amines.

3. Utilisation selon revendication 1, **caractérisée par le fait que**, pour l'amidation des groupes carboxyle des polysiloxanes, on a utilisé le 2-amino-2-méthyl-propanol-1, l'éthanolamine, la morpholine et/ou la triéthanolamine.

4. Utilisation selon l'une des revendications qui précèdent, **caractérisée par le fait que** les groupes carboxyle libres, non amidés, sont neutralisés en totalité ou en partie.

5. Utilisation selon l'une des revendications qui précèdent, **caractérisée par le fait que** les polysiloxanes à groupes carboxyle sont des diméthylpolysiloxanes.

6. Utilisation selon l'une des revendications qui précédent, en tant que produits pour les soins de la peau et de la chevelure.

7. Utilisation selon l'une des revendications qui précèdent, sous la forme d'émulsions.

8. Utilisation selon la revendication 6 ou 7, dans des shampooings.

9. Utilisation selon la revendication 6 ou 7, dans des fixateurs pour la chevelure.

10. Utilisation selon la revendication 6 ou 7, en tant que produit auxiliaire avant, durant ou après la teinture de la chevelure, la décoloration de la chevelure ou l'ondulation permanente.

11. Utilisation selon les revendications 1 à 5, pour les soins de la bouche.

12. Utilisation selon les revendications 1 à 5, pour les soins des ongles des mains et des pieds.

13. Compositions cosmétiques contenant des polysiloxanes à groupes carboxyle qui sont préparés à partir de diméthylpolysiloxanes ou de méthyl-propylpolysiloxanes à groupes terminaux anhydride succinique et dont les groupes anhydride ont ensuite été hydrolysés et neutralisés, les groupes carboxyle étant amidés en proportions de 0 à 50 mol %.
